Europäisches Patentamt

(19) European Patent Office

Office européen des brevets

(11) Publication number: **0 183 400**
**A2**

(12) # EUROPEAN PATENT APPLICATION

(21) Application number: 85307962.2

(22) Date of filing: 01.11.85

(51) Int. Cl.⁴: **A 61 M 5/14**

(30) Priority: 13.11.84 US 670525

(43) Date of publication of application:
04.06.86 Bulletin 86/23

(84) Designated Contracting States:
DE FR GB

(71) Applicant: SYBRON CORPORATION
1100 Midtown Tower
Rochester, NY 14604(US)

(72) Inventor: Langer, Alois A.
600 Woodside
Pittsburgh Pennsylvania 15221(US)

(72) Inventor: Rinne, Albert W.
2865 Cherry St.
Bethel Park Pennsylvania 19102(US)

(74) Representative: Oliver, Roy Edward et al,
POLLAK MERCER & TENCH High Holborn House 52-54
High Holborn
London WC1V 6RY(GB)

(54) Injector control.

(57) A control system for an angiographic injector precisely regulates the speed of a motor (M) used to expel contrast medium from a syringe, for instance. The control system is centered around a microprocessor (1) which receives commands from the operator via a key-board and then regulates the speed of the motor (M) by supplying drive command pulses to a circuit which integrates the difference in frequency between the command pulses and another pulse train whose frequency is proportional to actual motor speed. The control system also includes a velocity loop which uses the back EMF of the motor (M) to supply an analog voltage also proportional to motor speed to a difference amplifier (20) in a conventional velocity loop. The system is a dual loop controller where the second loop (F) provides approximate speed control, while the first loop (E) provides a "velocity correction" voltage required to bring the system to zero steady-state speed error. Motor speed pulses are provided by an incremental encoder (9,10), the pulses from which are counted by the microprocessor (1) to limit the volume of fluid injected. Various safety systems guard the injector's performance and help prevent over-volume injections. These systems are part of the velocity control and are also disclosed.

./...

FIG. 1

This invention relates to the general field of angiography, namely the technique for studying the vascular system by means of X-rays while injecting a "contrast medium", typically an iodine-based fluid, into the blood vessel or organ to be studied. In order for this process to be maximally successful, the flow from the syringe must be precisely controlled and this control has been the subject of much research and is described in several patent specifications. These known systems all exhibit various problems, most of which are eliminated by the system disclosed here. US-A-3623474 and US-A-3631847 disclose systems in which a signal directly proportional to flow rate is fed back to produce a flow error signal, which is then used to control motor speed. The disadvantages of straight velocity-control systems are discussed in US-A-3701345 and mainly concern the problems involved in measuring motor velocity over a wide range. For example, the velocity feedback system originally disclosed included a tachometer which produced an analog feedback voltage. This feedback scheme is later stated to be prone to drift and to be subject to noise at low speeds. A position feedback system for solving this problem is disclosed in US-A-3701345. In such a system, a potentiometer is used to sense the position of the syringe plunger. The position signal is compared to a command signal and the difference drives the motor. If the command is a ramp of constant slope, then the motor will move at nearly constant velocity. The position signal from the potentiometer is also used to limit the volume of fluid ejected from the syringe, by comparing it to a known position command signal which represents the position at which the injector should stop.

Even this improved control system has its limitations, however. Since a signal representing position is fed back, the system only guarantees that the average velocity between two positions is such that the plunger is in the right place at the end of a certain time interval. This means that velocity can increase and decrease around this average value as long as the average stays the same. From the standpoint of control system arrangement, there is an extra integrator in the feedback loop and system stability is compromised. In relation to performance accuracy in angiography, this implies that a position control system is not sufficiently resistant to factors which might cause brief changes in velocity. One such factor, which is quite significant, is represented by imperfections in the ballscrew mechanism, which is typically used to convert the rotary motion of the motor to the linear motion required to move the plunger. The present injector is of significantly greater power than previous machines by almost a factor of two. The force on the ballscrew is doubled and a larger ballscrew has to be used. This means that the possibility of velocity variations due to ballscrew effects is increased. The control system of the invention therefore addresses this problem by controlling velocity directly and incorporates a velocity correction feedback path to maintain velocity control with great accuracy.

Angiographic injectors typically employ a mechanical means for stopping the plunger in case of failure. Given the increased power of the system of the invention, such mechanical means would have to be extremely rugged, making the injector head very large and unwieldy. However, given the power of microprocessors to carry out performance tests at extremely high speed, it is possible to design an injector with microprocessor control and eliminate any mechanical means for limiting plunger travel. Given that many users do not use mechanical limits, that such limits are subject to great stress and may in fact fail themselves and that it is impossible to set them for each volume limit in a multi-bolus injection, the use of an "electronic stop" can in fact result in enhanced system reliability.

It is, therefore, an object of the present invention to provide an apparatus and a method for improved injection rate control in an angiographic injector and, in particular, an apparatus and a method yielding great system stability and being resistant to factors which may excite modes of short-term speed variations.

According to one aspect of this invention, an injector comprises a motor arranged to position a piston, characterised in that a modulator is connected both to a processor being arranged to produce a flow command signal and a first pulse train of frequency proportional to the flow command signal, and to the motor, for receiving the flow command signal and driving the motor at a rate proportional to the flow rate commanded by the flow command signal, the motor when so driven providing a feedback signal corresponding to the rate at which it is being driven by the modulator, and that the injector also includes pulse-producing means operated by the motor, for producing a second pulse train having a frequency proportional to the actual rate at which the motor positions the piston, and integrating means responsive to both of the pulse trains, for integrating the difference between the frequencies of the pulse trains and producing a rate-correction signal, the modulator responding to the flow command signal, the feedback signal and the rate-correction signal so as to cause the motor to position the piston accurately.

According to another aspect of this invention, an injector comprises a motor arranged to position a piston characterised by a control arranged to provide a flow command signal for the motor and including feedback means arranged to modify the command signal so as to decrease energization of the motor when it drives the piston at a rate exceeding the rate corresponding to the flow command and the control also including proportional braking means having a current source which responds to decrease in the energization by electrically loading the motor in proportion to the amount by which its speed exceeds a value corresponding to the flow command.

According to a further aspect of the invention, a method is provided for controlling an electronic motor so as to drive an injector and cause it to inject a predetermined quantity of material during a predetermined time period, the motor, in operation, producing a back electromotive force proportional to its speed, characterized in that at the beginning of the time period,

(1) electrical power is applied to the motor at a value which, if maintained from the beginning to the end of the time period, causes just the predetermined quantity to be injected during the time period,

(2) a first stream of pulses is applied to integrating means at a rate set independently of the motor but being the desired injection rate, and

(3) a second stream of pulses is applied to the integrating means ———— at a rate set by the motor and varying in proportion to the speed of the motor, the integrating means, in operation, producing a difference signal representing the difference between the total numbers of first and second stream pulses at any instant since the beginning of the time period,

(4) and in that, during the time period, the application of the electrical power to the motor is modified by means of a feedback signal which is representative of both the back emf and the difference signal, whereby the speed of the motor is regulated more accurately.

Since a microprocessor is used in the injector to enhance flexibility for injection parameter entry by the operator and to provide a means for sophisticated "electronic stop" volume limiting backup mechanisms, the invention also provides a control system which is easily commanded by a microprocessor.

Microprocessors by their nature cannot directly control in an analog fashion, such as has been the custom in the past. To be part of a system operating in an analog fashion with great precision requires a precise digital-to-analog converter, which is a very expensive and potentially unreliable component. Microprocessors are best suited to supplying some sort of pulse, perhaps with the pulse interval representing the variable to be controlled. It is, therefore, an object of this invention to provide a velocity control system which is commanded by the natural output of a computer, namely, a pulse train.

Computers have one potential drawback, however, because they are subject to "crashing". This means in general that the processor fails to execute its programme in a controlled manner and, in fact, may be running useless code. A "crashed" computer may do unpredictable things and it is therefore also an object of this invention to provide circuits which cause the motor not to run, in the case of microprocessor failure.

Due to the nature of the process of injecting fluid into the body, it is critical that the machine should stop, if any abnormal operating condition arises. The parameters which need to be watched are flow, volume and pressure. It is, therefore, another object of this invention to provide redundant sensors for these critical parameters and to provide backup circuits for controlling them, should a failure occur. Furthermore, should these backups become ineffective, it is an additional object of the invention to provide error-detecting circuits for stopping the machine.

Preparing a patient for angiography is a significant procedure. A catheter is introduced into a peripheral artery or vein or other vascular element and therefore the process is invasive. Should the injector fail, all preparation of the patient might be in vain and therefore it is an additional object of the invention to provide a self-test feature, so that whether or not the machine is operational can be determined before it is asked to perform an injection. This self-test feature is activated when the machine is turned on and is very thorough and extensive.

Basically, the present invention is an improvement over the prior art because it employs dual loop control. One of the loops is very precise and is under the direct control of the microcomputer. Plunger position is tracked by two independent measuring circuits, an incremental encoder and a potentiometer. Since volume is such a critical parameter, these components are not arranged as a primary circuit and a backup circuit, but rather are constantly and simultaneoeously monitored by the computer. Both are measures of injected volume and must agree in order for the injection to continue. Thus, both of the volume-measuring circuits must be operational or else the machine stops. The incremental encoder is based upon a wheel attached to the motor shaft and gives, say, 32 pulses per revolution of the motor. These pulses are counted by the computer and are used to limit the injection volume. The precision of this count is such that 0.1 ml volumes are possible, which is an accuracy heretofore unavailable in the injector field. In keeping with the philosophy of maximum redundancy on volume limitation, an additional circuit monitors the voltage from the potentiometer and transmits an error interrupt command to the computer, should the plunger somehow move too far forward in spite of the other volume-limiting systems.

These and other aspects of the invention will become evident to those skilled in the art from the following description of certain preferred embodiments, given in conjunction with the accompanying drawings; in these:

Figure 1 shows a block diagram of a preferred form of the overall system;

Figures 2 and 3 show, as additional block diagrams, components of the overall system in greater detail.

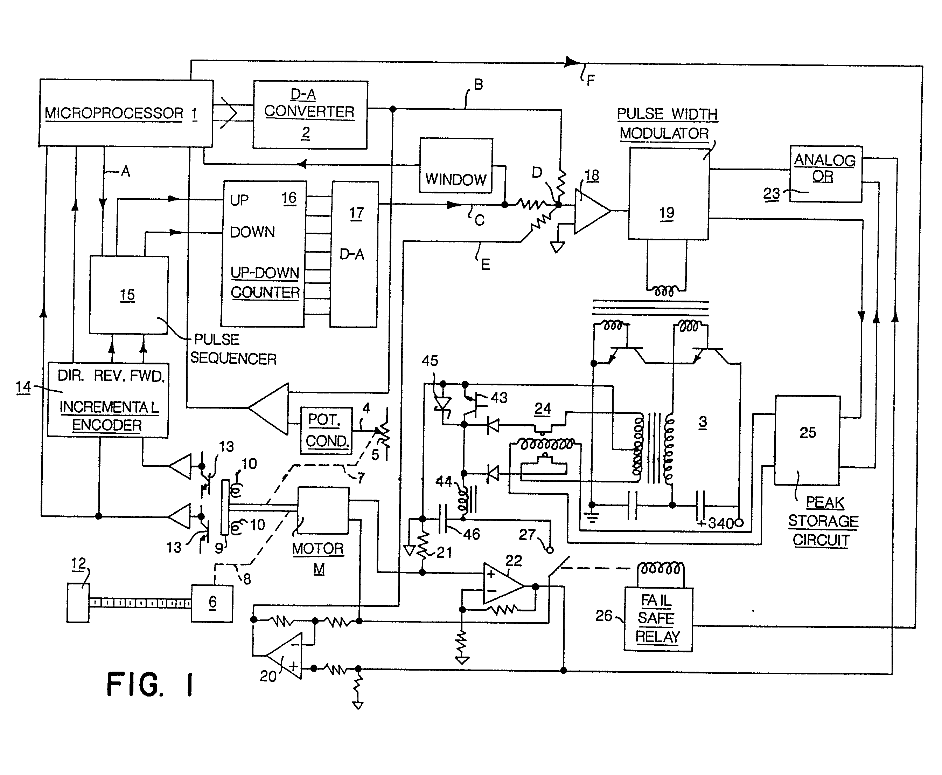

Figure 1 shows an overall view of the injector and its velocity control system. Central to the control is a microprocessor 1, which communicates with a removable console (not shown) via a serial link, as is common in computer systems. By actuating keys on the console, various parameters necessary to complete an injection can be entered by the user.

Upon receiving a valid flow rate command and a signal to start the injector, the microprocessor supplies a pulse train, in a line A, whose frequency is directly proportional to the desired flow rate. The frequency can be very accurately controlled by known software methods and is derived from a quartz crystal clock included in the processor 1. Quartz crystals are, of course, known for their frequency, accuracy and stability, contributing to the accuracy of the flow command pulse train. At the same time, the microprocessor 1 sends an 8-bit flow command word to a multichannel D-A converter 2. The resulting low precision analog voltage becomes the command for a velocity loop B. This need not be of high precision, because of a velocity correction loop C which in effect "trims" the velocity loop B, as will be seen subsequently. Therefore, there are two variables which set the motor speed, the voltage from the DAC 2 and the frequency of the pulse train.

At the other end of the control system is a power amplifier 3, which supplies the high power, often in excess of 1000 watts, necessary to drive the motor M. This amplifier 3 is of conventional design and is a switching amplifier derived from the half bridge topology. The main components are illustrated, but are not described in great detail herein.

The motor M drives the slider 4 of a potentiometer 5, a reduction gear 6 (the driving means being represented by dashed line ganging 7 and 8) and an encoder or interrupter wheel 9 on the shaft of the motor M. The encoder wheel 9 interrupts light beams from two LEDs 10, or the like, and the reduction gear 6 drives a lead screw 11 which, when rotated, in turn advances a piston 12 along the length of the screw 11.

Except for the interruptions due to the wheel 9, the LED light beams illuminate respective light-sensitive transistors 13. The interruptions result in pulses from each of the two channels being applied by the transistors 13, which are approximately 90° out of phase, to an incremental encoder circuit 14 having three outputs, shown as DIR, REV and FWD. The encoder 14 assigns direction significance to the pulses, i.e., whether they result from clockwise or anti-clockwise motor rotation, i.e., the lead screw 11 goes forward or in reverse, so that one of the three outputs, FWD, is for forward pulses and another, REV, for reverse pulses. The third output, DIR, is for direction. Total pulses are also sent to the processor 1, from one direct channel of the wheel 9, whereas the other two outputs are applied to a pulse sequencer 15, as are the pulses on line A from the processor 1. Total pulses are counted by the processor 1 to limit volume.

The pulse sequencer itself is connected to an up-down counter 16, causing the latter to count both the line A pulses and reverse pulses from the incremental encoder. On the other hand, the sequencer 15 makes the up-down counter count down forward pulses. At any given moment, the parallel output of the up-down counter 16 has a word which represents the algebraic difference then existing between the net pulse output of the processor 1, via line A, and the net pulse output of the interrupter wheel 9. This word is applied to a D-A converter 17, which produces an analog signal, which is algebraically summed at a terminal D with the flow command signal by an amplifier 18, which applies the resultant signal to a pulse width modulator 19. The modulator 19 increases (or decreases) the duty cycle of pulses fed to the motor M, thus varying the power of the motor M, as is common in off-time switching of power supplies.

Via a conventional velocity loop E, an analog signal from an amplifier 20 representing motor current is summed at the terminal D with the flow command and velocity correction signal. Motor voltage or back emf is roughly proportional to motor speed. Motor current flowing through a resistor 21 creates a voltage which is sensed by an amplifier 22, the output voltage of which is applied to the amplifier 20 and to an analog OR ciruit 23. A signal proportional to current is subtracted from the motor voltage to compensate for motor armature resistance. Another measure of motor current is derived from a current-sensing transformer 24, by means of a peak storage circuit 25, and the latter applies this measure as an analog voltage to the modulator 19 and to the OR circuit 23, which in turn applies the larger of the current-derived voltage of the amplifier 22 and the storage circuit voltage of 25 to the modulator 19, thereby limiting the injection pressure.

At any instant from the beginning time of the pulse train on the line A, the pulse total represents how much liquid should have been injected by that time. At the same time, the net count of forward and reverse pulses from the decoder 17 represents the actual amount injected since the motor M began to rotate the wheel 9. Therefore, any non-zero output of the up-down counter 16 is the measure of an error in injected amount and, by summing that non-zero output with the flow command, the latter is increased or decreased, as needed, to compensate for the injection error.

Preferably, the processor 1 also monitors volume error by comparing the piston-position proportional voltage at the potentiometer 5 and an absolute volume limit command signal from another channel of the D-A converter 17 and shuts off the motor M if an intolerable discrepancy develops.

As suggested above, a number of conditions can occur which call for stopping the injector for the good of the patient. However, it has also been noted that the motor M is rather powerful, so stopping it can be a problem. According to another preferred feature of this invention, the motor M stops in response to proportional braking, a technique which optimizes the application of braking effect to the motor M.

Also, under some circumstances, it is desired to ramp the flow command downward, i.e., decrease the motor speed steadily and not abruptly, but nevertheless rather markedly. In the prior art, while the pressure in the cartridge aids the ramp process to some extent, the motor nevertheless tends to coast, due to armature inertia, i.e., to resist being slowed down, so to speak. It is to be noted that the duty cycle control of the motor acts by decreasing the energization of the motor, but cannot reverse it.

According to an aspect of the invention, ramping of the motor is provided for braking the motor speed in proportion to the desired rate of decrease, as represented by decreasing the flow command in accordance with the desired downward ramp slope.

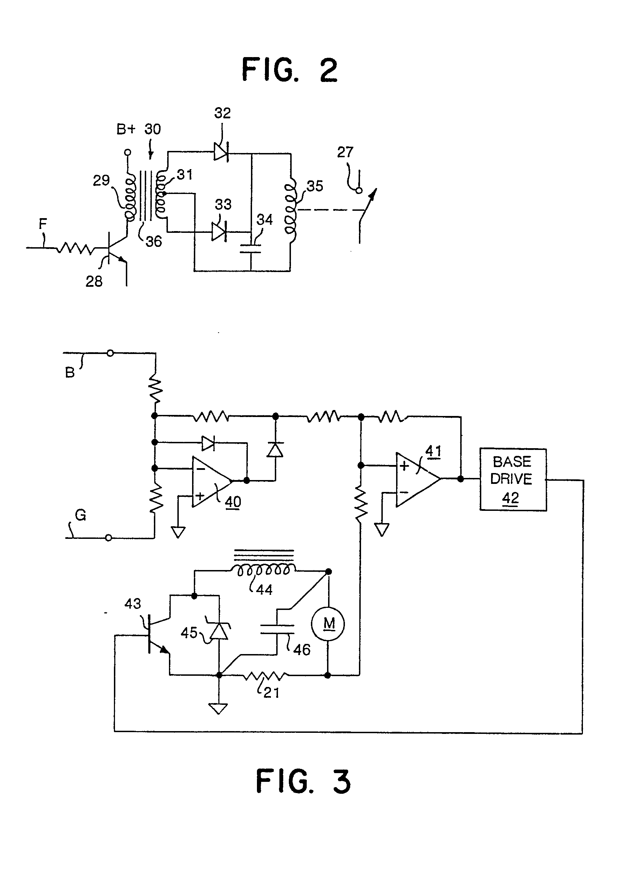

When the motor M is not being supplied with driving current, it is coasting and therefore being a generator. Thus, in Figure 3, the flow command positive voltage on the line B is applied to the negative terminal of an amplifier 40, where it is summed with a motor parameter negative voltage derived from line G. The latter voltage represents actual flow, so if the motor speed is greater than its command value the amplifier 40 will produce a flow difference positive output voltage, which will turn on the amplifier 41. The latter has its positive terminal connected both to the flow difference voltage and to the motor current representing voltage at the connection of a resistor 21 to the motor M and turns on a base drive circuit 42 for a transistor 43. At this time, if the power amplifier 3 is off, effectively it does not exist, and what the motor M sees is a short circuit at the transistor 43. As the motor M is coasting at this point, it is acting as a generator loaded by the transistor 43 to the extent premitted by the base drive circuit 42. At the same time, current through an inductor 44 and the motor M increases in linear fashion, due to their inductance, and the voltage applied by the resistor 21 to the amplifier 41 becomes negative and turns off the amplifier 41. During the flyback period of the inductor 44, while current flows through a zener clamp 45, the motor current decreases, eventually turning on the amplifier 41 again.

The above-described cycle repeats and as the flow command is decreasing, energy will be transferred from the motor M to the zener 45 at the rate required to slow the motor M.

A fail-safe relay is desirably provided to open a contact 27, should the processor pulse output on the line F fail. Thus, as shown in Figure 1, the pulses are applied via the line F to the base of a transistor 28, so as to turn the transistor 28 on and off at the pulse frequency. When the transistor 28 is on, there is a current due to the D.C. supply voltage at a B+ terminal. This current passes through the primary winding 29 of a transformer 30 also having a tapped winding 31, at the ends of which are steering diodes 32 and 33. The anodes of the diodes 32,33 are connected together and to one side of a capacitor 34 and to one end of a relay operating coil 35 which when energized closes the contact 27, the other end of the coil 35 being connected to the other side of the capacitor 34 and to the tap on the winding 31. The transformer 30 has a core 36 which is constructed so as to store as much energy therein as is coupled through it when the transistor 28 is turned on by a pulse from the line F. The energy storage capacity of the core 36 is a function mainly of the size of an air gap in the core 36 and the number of turns and, by appropriately proportioning these parameters, it is possible to make the energy stored in the core 36 substantially equal to that transferred through it when the transistor 28 is pulsed on.

Since the motor M is driven by the current in the loop containing the contact 27, this contact must be closed for the motor M to turn. Due to the "flyback" effect, current flows substantially continuously through the coil 35, i.e., when the transistor 28 turns off between pulses, the energy stored in the core 36 provides current in the coil 35.

CLAIMS:

1. An injector comprising a motor (M) arranged to position a piston (12),
characterised in that
a modulator (19) is connected both to a processor (1) being arranged to produce a flow command signal and a first pulse train of frequency proportional to the flow command signal, and to the motor (M), for receiving the flow command signal and driving the motor (M) at a rate proportional to the flow rate commanded by the flow command signal, the motor (M) when so driven providing a feedback signal corresponding to the rate at which it is being driven by the modulator (19), and that the injector also includes pulse-producing means (9,10) operated by the motor (M), for producing a second pulse train having a frequency proportional to the actual rate at which the motor (M) positions the piston (12), and integrating means (18-25) responsive to both of the pulse trains, for integrating the difference between the frequencies of the pulse trains and producing a rate-correction signal, the modulator (19) responding to the flow command signal, the feedback signal and the rate-correction signal so as to cause the motor (M) to position the piston accurately.

2. An injector comprising a motor (M) arranged to position a piston (12),
characterised by a control (40-46) arranged to provide a flow command signal for the motor (M) and including feedback means arranged to modify the command signal so as to decrease energization of the motor (M) when it drives the piston (12) at a rate exceeding the rate corresponding to the flow command and the control (40-46) also including proportional braking means (45) having a current source (42) which responds to decrease in the energization by electrically loading the motor (M) in proportion to the amount by which its speed exceeds a value corresponding to the flow command.

3. An injector system having a pulse-commanded motor (M), a source (9-15) of command pulses and a modulator (19) connected in circuit with the motor (M) for driving it as a function of the pulses, characterised in that

the circuit includes an open contact (27) which must be kept closed in order for the motor (M) to be driven and that the system also includes fail-safe means (28-36) responsive to the pulses to keep the contact (27) closed.

4. A relay driver comprising a transformer (30) having a first winding (29), a second winding (31) and a core (36) coupling the windings (29,31), characterised in that

the core (36) is constructed and arranged to return to the second winding (31) substantially all the energy stored in the core (36) in response to termination of a pulse of current in the first winding (29) and that steering diode means (32,33) are provided for causing current in the second winding (31), due to the pulse, and current in the second winding (31), due to the stored energy, to have the same sense, whereby connection of a D.C. load (35) across the second winding (31), pulsing of the first winding (29) nevertheless cause the load (35) to be substantially continuously energized.

5. A relay driver according to claim 4, wherein a relay solenoid (35) is connected across the second winding (31), a first diode (32) interconnects one end of the second winding (31) and one end of the solenoid (35), the second diode (33) interconnects an intermediate point on the second winding (31) and the junction of the first diode (32) and such one end of the solenoid (35) and the other ends of the solenoid (35)

0183400

and the second winding (31) are directly interconnected.

6. A method of controlling an electric motor (M) so as to drive an injector and cause it to inject a predetermined quantity of material during a predetermined time period, the motor (M), in operation, producing a back electromotive force proportional to its speed, characterised in that,
at the beginning of the time period,

(1) electrical power is applied to the motor (M) at a value which, if maintained from the beginning to the end of the time period, causes just the predetermined quantity to be injected during the time period,

(2) a first stream of pulses is applied to integrating means (18-25) at a rate set independently of the motor (M) but being the desired injection rate, and

(3) a second stream of pulses is applied to the integrating means (18-25) at a rate set by the motor (M) and varying in proportion to the speed of the motor (M), the integrating means (18-25), in operation, producing a difference signal representing the difference between the total numbers of first and second stream pulses at any instant since the beginning of the time period,

(4) and in that, during the time period, the application of the electrical power to the motor (M) is modified by means of a feedback signal which is representative of both the back emf and the difference signal, whereby the speed of the motor (M) is regulated more accurately.

FIG. 1

0183400

0183400

FIG. 2

FIG. 3